# EUROPEAN PATENT SPECIFICATION

(11) **EP 3 824 914 B1**
(45) Date of publication and mention of the grant of the patent: **29.04.2026**
(21) Application number: 20203926.9
(22) Date of filing: 26.10.2020
(51) Int. Cl.: A61L 29/04, A61L 31/04

(54) **MEDICAL RUBBER COMPOSITION AND MEDICAL RUBBER COMPONENT**
MEDIZINISCHE GUMMIZUSAMMENSETZUNG UND MEDIZINISCHE KAUTSCHUKKOMPONENTE
COMPOSITION DE CAOUTCHOUC MÉDICAL ET COMPOSANT DE CAOUTCHOUC MÉDICAL

(30) Priority: 19.11.2019 JP 2019209030
(43) Date of publication of application: 26.05.2021
(73) Proprietor: Sumitomo Rubber Industries, Ltd., Kobe-shi, Hyogo-ken 651-0072 (JP)
(72) Inventor: MATSUTANI, Yuichiro, Kobe-shi, Hyogo 651-0072 (JP); YAMAMOTO, Keishi, Kobe-shi, Hyogo 651-0072 (JP); INOUE, Toshishige, Kobe-shi, Hyogo 651-0072 (JP)
(74) Representative: Manitz Finsterwald Patent- und Rechtsanwaltspartnerschaft mbB

(56) References cited:
- US-A1- 2003 100 696
- US-A1- 2013 137 825

## Description

### FIELD OF THE INVENTION

The present invention relates to a medical rubber composition and a medical rubber component, more specifically relates to a technology for improving gas permeation resistance of a medical rubber component.

### DESCRIPTION OF THE RELATED ART

A medical rubber product for a pharmaceutical container is required to have high quality properties and physical properties. For example, qualities and properties required for a medical rubber stopper sealing or plugging an opening of a vial storing a formulation of antibiotics or the like should comply with the infusion rubber stopper test of Japanese Pharmacopoeia, 17^{th} Edition. Further, several items such as gas permeation resistance, non-elution, high cleanability, chemical resistance, needle penetration resistance, self-sealability and high slidability are required for a medical rubber stopper sealing an opening of a vial.

For example, JP 2013-112703 A discloses a thermoplastic elastomer composition used for a medical rubber product, including a dynamically crosslinked butyl rubber and a thermoplastic resin, and having the thermoplastic resin in an amount of 8 mass % or less.

JP 2001-340425 A discloses a medical rubber stopper applied to an opening of a medical container and having a penetration portion through which an injection needle of an injector can be penetrated, wherein a nylon film layer with a thickness of from 20 to 200 µm is provided on a top face of the medical rubber stopper.

JP 2015-62564 A discloses a medical rubber member, obtained by press molding a stacked sheet of an unvulcanized rubber mainly containing a rubber for forming a liquid-contacting portion and an unvulcanized rubber mainly containing a butyl rubber for forming a non-liquid-contacting portion, wherein a color difference dE between the liquid-contacting portion and the non-liquid-contacting portion is 6.0 or more in NBS units.

JP 3193895 B discloses a rubber stopper for a pharmaceutical container, obtained by vulcanizing a halogenated butyl rubber blended with a super high molecular weight polyethylene fine powder in an amount of from 5 to 25 parts by weight relative to 100 parts by weight of the halogenated butyl rubber, without the presence of a zinc compound and using at least one kind of 2-substitute-4,6-dithiol-s-triazine derivative or an organic peroxide.

US 2013/137825 A1 discloses a thermoplastic elastomer composition comprising a dynamically-crosslinked butyl rubber and a thermoplastic resin; and having a thermoplastic resin content of not more than 8% by mass.

### SUMMARY OF THE INVENTION

A product formed by crosslinking a rubber composition containing a butyl rubber is used for a medical rubber product, in light of its excellent gas permeation resistance and the like. However, there is room for improvement in gas permeation resistance of a medical rubber product formed by crosslinking a rubber composition containing a conventional butyl rubber.

The present invention has been made in view of the above problems. An object of the present invention is to provide a medical rubber composition providing excellent gas permeation resistance. Another object of the present invention is to provide a medical rubber component having excellent gas permeation resistance.

The present invention provides a medical rubber composition containing (a) a base polymer containing a halogenated butyl rubber, and (b) a liquid polymer, wherein the medical rubber composition contains (b) the liquid polymer in an amount of from 1 part by mass to 18 parts by mass with respect to 100 parts by mass of (a) the base polymer. Further, (b) the liquid polymer contains a liquid polybutene or a liquid polybutadiene. The present invention further provides a medical rubber member formed from the medical rubber composition according to the present invention. The present invention has been accomplished based on the findings of the inventors that blending (b) the liquid polymer in (a) the base polymer containing the halogenated butyl rubber instead of an ordinary oil improves gas permeation resistance of the obtained medical rubber component.

If the medical rubber composition according to the present invention is used, a medical rubber product having excellent gas permeation resistance is obtained.

### BRIEF DESCRIPTION OF THE DRAWINGS

Fig. 1 is a figure illustrating an embodiment of the medical rubber component according to the present invention (plunger stopper);
Fig. 2 is a figure illustrating an embodiment of the medical rubber component according to the present invention (rubber stopper);
Fig. 3 is a figure illustrating an embodiment of the medical rubber component according to the present invention (rubber stopper for a vial);
Fig. 4 is a figure illustrating an embodiment of the medical rubber component according to the present invention (nozzle cap); and
Fig. 5 is a figure illustrating an embodiment of the medical rubber component according to the present invention (rubber stopper for a vacuum blood collecting tube).

### DESCRIPTION OF THE PREFERRED EMBODIMENT

The medical rubber composition according to the present invention contains (a) a base polymer containing a halogenated butyl rubber and (b) a liquid polymer, wherein the medical rubber composition contains (b) the liquid polymer in an amount of from 1 part by mass to 18 parts by mass with respect to 100 parts by mass of (a) the base polymer.

First, (a) the base polymer containing the halogenated butyl rubber will be explained. Examples of the halogenated butyl rubber include a chlorinated butyl rubber, a brominated butyl rubber, and a bromide of a copolymer of isobutylene and p-methylstyrene. As the halogenated butyl rubber, the chlorinated butyl rubber or the brominated butyl rubber is preferable. The chlorinated butyl rubber or brominated butyl rubber is, for example, obtained by an addition reaction or substitution reaction between chlorine or bromine and an isoprene moiety in a butyl rubber, specifically a double bond and/or a carbon atom adjacent to a double bond of the isoprene moiety. It is noted that the butyl rubber is a copolymer obtained by polymerizing isobutylene and a small amount of isoprene.

It is noted that (a) the base polymer containing the halogenated butyl rubber differs from (b) the liquid polymer in the point that (a) the base polymer containing the halogenated butyl rubber is a solid at normal temperature (23 °C).

The amount of halogen in the halogenated butyl rubber is preferably 0.5 mass % or more, more preferably 1 mass % or more, and even more preferably 1.5 mass % or more, and is preferably 5 mass % or less, more preferably 4 mass % or less, and even more preferably 3 mass % or less.

Specific examples of the chlorinated butyl rubber include at least one member selected from CHLOROBUTYL 1066 (stabilizer: NS, halogen content: 1.26%, Mooney viscosity: 38 ML₁₊₈ (125 °C), specific gravity: 0.92) available from Japan Butyl Co. Ltd. and LANXESS X_BUTYL CB1240 available from LANXESS Co. Ltd.

Specific examples of the brominated butyl rubber include at least one member selected from BROMOBUTYL 2255 (stabilizer: NS, halogen content: 2.0%, Mooney viscosity: 46 ML₁₊₈ (125 °C), specific gravity: 0.93) available from Japan Butyl Co. Ltd. and LANXESS X_BUTYL BBX2 available from LANXESS Co. Ltd.
(a) The base polymer may further contain a rubber component other than the halogenated butyl rubber. Examples of the other rubber component include butyl rubber, isoprene rubber, butadiene rubber, styrene-butadiene rubber, natural rubber, chloroprene rubber, nitrile rubber such as acrylonitrile-butadiene rubber, hydrogenated nitrile rubber, norbornene rubber, ethylene-propylene rubber, ethylene -propylene-diene rubber, acrylic rubber, ethylene-acrylate rubber, fluororubber, chlorosulfonated polyethylene rubber, epichlorohydrin rubber, silicone rubber, urethane rubber, polysulfide rubber, phosphazene rubber, and 1,2-polybutadiene. These rubber components may be used solely, or two or more of them may be used in combination.

In case of using the other rubber components, the amount of the halogenated butyl rubber in (a) the base polymer is preferably 90 mass % or more, more preferably 95 mass % or more, and even more preferably 98 mass % or more. In addition, it is also preferable that (a) the base polymer consists of the halogenated butyl rubber.

### (b) Liquid polymer

Next, (b) the liquid polymer used in the present invention will be explained. (b) The liquid polymer is a liquid polymer at the room temperature (23 °C). In accordance with the invention (b) the liquid polymer includes liquid polybutene or liquid polybutadiene. Among them, the liquid polybutene is more preferable.

The liquid polybutene is not particularly limited, as long as it is a polymer primarily composed of butene, and examples thereof include a polymer having 1-butene as its main constituent component, and a polymer having isobutene as its main constituent component (liquid polyisobutene). In the present invention, as the liquid polybutene, the liquid polyisobutene is preferably used.

As the liquid polybutene, a hydrogenated product of the polybutene (hydrogenated liquid polybutene) may also be used.

(b) The liquid polymer may be used solely, or two or more of them may be used in combination.

The dynamic viscosity of (b) the liquid polymer at the temperature of 40 °C is preferably 900 mm²/s or more, more preferably 2000 mm²/s or more, and even more preferably 3000 mm²/s or more, and is preferably 100000 mm²/s or less, more preferably 50000 mm²/s or less, and even more preferably 30000 mm²/s or less. If the dynamic viscosity at the temperature of 40 °C falls within the above range, a better rubber can be obtained without bleeding of the low molecular weight component and problem on the process.

The dynamic viscosity of (b) the liquid polymer at the temperature of 100 °C is preferably 50 mm²/s or more, more preferably 60 mm²/s or more, even more preferably 70 mm²/s or more, and most preferably 150 mm²/s or more, and is preferably 4000 mm²/s or less, more preferably 3000 mm²/s or less, and even more preferably 2000 mm²/s or less. If the dynamic viscosity at the temperature of 100 °C falls within the above range, a better rubber can be obtained without bleeding of the low molecular weight component and problem on the process.

The dynamic viscosity of (b) the liquid polymer is a value measured at the temperature of 100 °C or 40 °C according to JIS K2283-2000.

The number average molecular weight of (b) the liquid polymer is preferably 650 or more, more preferably 700 or more, and even more preferably 750 or more, and is preferably 2800 or less, more preferably 2500 or less, and even more preferably 2000 or less. It is noted that the measurement of the number average molecular weight is conducted by gel permeation chromatography ("HLC-8120GPC", available from Tosoh Corporation) using a differential refractometer as a detector under the conditions of column: GMHHXL (available from Tosoh Corporation), column temperature: 40 °C, and mobile phase: tetrahydrofuran, and calculated by converting based on polystyrene standard.

The amount of (b) the liquid polymer in the medical rubber composition according to the present invention is preferably 1 part by mass or more, more preferably 3 parts by mass or more, and even more preferably 5 parts by mass or more, and is preferably 18 parts by mass or less, and even more preferably 15 parts by mass or less, with respect to 100 parts by mass of (a) the base polymer component. If the amount of (b) the liquid polymer falls within the above range, a rubber having lower gas permeability and better processability can be obtained.

The medical rubber composition according to the present invention preferably contains (c) a crosslinking agent. (c) The crosslinking agent is blended for crosslinking the halogenated butyl rubber component contained in (a) the base polymer. (c) The crosslinking agent is not particularly limited, as long as it is a crosslinking agent capable of crosslinking the halogenated butyl rubber. Examples of (c) the crosslinking agent include sulfur, a metal oxide, a resin crosslinking agent, an organic peroxide, and a triazine derivative. These crosslinking agents may be used solely, or two or more of them may be used in combination.

Examples of the sulfur used as the crosslinking agent include powder sulfur, fine powder sulfur, precipitated sulfur, colloidal sulfur, and chlorinated sulfur.

Examples of the metal oxide used as the crosslinking agent include magnesium oxide, calcium oxide, zinc oxide, and copper oxide.

Examples of the resin crosslinking agent include alkyl phenol formaldehyde resins such as an alkyl phenol formaldehyde resin, a thermo-reactive phenol resin, a phenol dialcohol-based resin, a bisphenol resin, and a thermo-reactive bromomethylalkylated phenol resin.

Specific examples of the organic peroxide include a dialkyl peroxide, a peroxy ester, a peroxy ketal, and a hydroperoxide. Examples of the dialkyl peroxide include di(2-t-butylperoxyisopropyl) benzene, dicumyl peroxide, 2,5-dimethyl-2,5-di(t-butylperoxy) hexane, t-butylcumyl peroxy, di-t-hexylperoxy, di-t-butylperoxy, and 2,5-dimethyl-2,5-di(t-butylperoxy) hexyne-3. Examples of the peroxy ester include t-butylperoxy maleate, t-butylperoxy-3,3,5-trimethyl cyclohexanoate, t-butylperoxy laurate, t-butylperoxyisopropyl monocarbonate, t-hexylperoxy benzoate, 2,5-dimethyl-2,5-di(benzoylperoxy) hexane, t-butylperoxy acetate, and t-butylperoxy benzoate. Examples of the peroxy ketal include 1,1-di(t-hexylperoxy)-3,3,5-trimethylcyclohexane, 1,1-di(t-hexylperoxy) cyclohexane, 1,1-di(t-butylperoxy)-2-methylcyclohexane, 1,1-di(t-butylperoxy) cyclohexane, 2,2-di(t-butylperoxy) butane, n-butyl-4,4-di(t-butylperoxy) valerate, and 2,2-di(4,4-di(t-butylperoxy) cyclohexyl) propane. Examples of the hydroperoxide include p-menthane hydroperoxide, and diisopropylbenzene hydroperoxide. These organic peroxides may be used solely, or two or more of them may be used in combination.

Examples of the triazine derivative used as the crosslinking agent include a compound represented by the general formula (1). 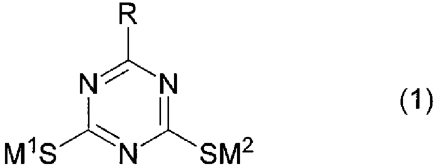 [In the formula, R is -SH, -OR¹, -SR², -NHR³ or -NR⁴R⁵ (R¹, R², R³, R⁴ and R⁵ represent an alkyl group, an alkenyl group, an aryl group, an aralkyl group, an alkylaryl group or a cycloalkyl group, and R⁴ and R⁵ may be identical to or different from each other.); M¹ and M² are H, Na, Li, K, 1/2Mg, 1/2Ba, 1/2Ca, an aliphatic primary amine, secondary amine or tertiary amine, a quaternary ammonium salt or a phosphonium salt; and M¹ and M² may be identical to or different from each other.]

In the general formula (1), examples of the alkyl group include an alkyl group having 1 to 12 carbon atoms such as methyl group, ethyl group, n-propyl group, isopropyl group, n-butyl group, isobutyl group, tert-butyl group, n-pentyl group, isopentyl group, tert-pentyl group, n-hexyl group, 1,1-dimethylpropyl group, octyl group, isooctyl group, 2-ethylhexyl group, decyl group, and dodecyl group. Examples of the alkenyl group include an alkenyl group having 1 to 12 carbon atoms such as vinyl group, allyl group, 1-propenyl group, isopropenyl group, 2-butenyl group, 1,3-butadienyl group, and 2-pentenyl group. Examples of the aryl group include a monocyclic or condensed polycyclic aromatic hydrocarbon group, and specific examples thereof include an aryl group having 6 to 14 carbon atoms such as phenyl group, naphthyl group, anthryl group, phenanthryl group, and acenaphthylenyl group. Examples of the aralkyl group include an aralkyl group having 7 to 19 carbon atoms such as benzyl group, phenethyl group, diphenylmethyl group, 1-naphthylmethyl group, 2-naphthylmethyl group, 2,2-diphenylethyl group, 3-phenylpropyl group, 4-phenylbutyl group, 5-phenylpentyl group, 2-biphenylylmethyl group, 3-biphenylylmethyl group, and 4-biphenylylmethyl group. Examples of the alkylaryl group include an alkylaryl group having 7 to 19 carbon atoms such as tolyl group, xylyl group, and octylphenyl group. Examples of the cycloalkyl group include a cycloalkyl group having 3 to 9 carbon atoms such as cyclopropyl group, cyclobutyl group, cyclopentyl group, cyclohexyl group, cycloheptyl group, cyclooctyl group and cyclononyl group.

Specific examples of the triazine derivative represented by the general formula (1) include 2,4,6-trimercapto-s-triazine, 2-methylamino-4,6-dimercapto-s-triazine, 2-(n-butylamino)-4,6-dimercapto-s-triazine, 2-octylamino-4,6-dimercapto-s-triazine, 2-propylamino-4,6-dimercapto-s-triazine, 2-diallylamino-4,6-dimercapto-s-triazine, 2-dimethylamino-4,6-dimercapto-s-triazine, 2-dibutylamino-4,6-dimercapto-s-triazine, 2-di(iso-butylamino)-4,6-dimercapto-s-triazine, 2-dipropylamino-4,6-dimercapto-s-triazine, 2-di(2-ethylhexyl) amino-4,6-dimercapto-s-triazine, 2-dioleylamino-4,6-dimercapto-s-triazine, 2-laurylamino-4,6-dimercapto-s-triazine, 2-anilino-4,6-dimercapto-s-triazine, and sodium salt or disodium salt thereof.

Among them, 2,4,6-trimercapto-s-triazine, 2-dialkylamino-4,6-dimercapto-s-triazine, and 2-anilino-4,6-dimercapto-s-triazine are preferable, and 2-dibutylamino-4,6-dimercapto-s-triazine is most preferable in view of easy availability.

In the present invention, the triazine derivative may be used solely, or two or more of the triazine derivatives may be used in combination.

Since the chlorinated butyl rubber and the brominated butyl rubber have different crosslinking mechanisms, a suitable crosslinking component is preferably chosen and used for the crosslinking. The medical rubber composition according to the present invention preferably contains the triazine derivative as (c) the crosslinking agent when containing the chlorinated butyl rubber as the halogenated butyl rubber. In addition, the medical rubber composition according to the present invention preferably contains the metal oxide as (c) the crosslinking agent when containing the brominated butyl rubber as the halogenated butyl rubber.

The amount of (c) the crosslinking agent in the medical rubber composition according to the present invention is preferably 0.2 part by mass or more, more preferably 0.4 part by mass or more, and even more preferably 0.6 part by mass or more, and is preferably 20 parts by mass or less, more preferably 15 parts by mass or less, and even more preferably 10 parts by mass or less, with respect to 100 parts by mass of (a) the base polymer component. If the amount of (c) the crosslinking agent falls within the above range, a rubber having better rubber properties (hardness, tension, Cset) and better processability (little burn) can be obtained.

In case of using the chlorinated butyl rubber as the halogenated butyl rubber and using the triazine derivative as (c) the crosslinking agent, the amount of (c) the crosslinking agent in the medical rubber composition is preferably 0.2 part by mass or more, more preferably 0.4 part by mass or more, and even more preferably 0.6 part by mass or more, and is preferably 4 parts by mass or less, more preferably 3 parts by mass or less, and even more preferably 2 parts by mass or less, with respect to 100 parts by mass of (a) the base polymer component. If the amount of (c) the crosslinking agent falls within the above range, a rubber having better rubber properties (hardness, tension, Cset) and better processability (little burn) can be obtained.

In case of using the brominated butyl rubber as the halogenated butyl rubber and using the metal oxide as (c) the crosslinking agent, the amount of (c) the crosslinking agent in the medical rubber composition is preferably 1 part by mass or more, more preferably 1.5 parts by mass or more, and even more preferably 2 parts by mass or more, and is preferably 20 parts by mass or less, more preferably 15 parts by mass or less, and even more preferably 10 parts by mass or less, with respect to 100 parts by mass of (a) the base polymer component. If the amount of (c) the crosslinking agent falls within the above range, a rubber having better rubber properties (hardness, tension, Cset) and better processability (little burn) can be obtained.

The medical rubber composition according to the present invention preferably does not contain a vulcanization accelerator. This is because the vulcanization accelerator may remain in the final rubber product, and dissolve into the pharmaceutical liquid in a syringe or vial. Examples of the vulcanization accelerator include a guanidine accelerator (e.g. diphenylguanidine), a thiuram accelerator (e.g. tetramethylthiuram disulfide, tetramethylthiuram monosulfide), a dithiocarbamate accelerator (e.g. zinc dimethyldithiocarbamate), a thiazole accelerator (e.g. 2-mercaptobenzothiazole, dibenzothiazyl disulfide), and a sulfonamide accelerator (e.g. N-cyclohexyl-2-benzothiazole sulfenamide, N-t-butyl-2-benzothiazole sulfenamide).

The medical rubber composition according to the present invention may contain a hydrotalcite. The hydrotalcite functions as an anti-scorching agent when crosslinking the halogenated butyl rubber, and also functions as an agent to prevent increase in compression permanent strain of the medical rubber member. Further, the hydrotalcite also functions as an acid acceptor to absorb chlorine-based gas or bromine-based gas happening when crosslinking the halogenated butyl rubber and prevent occurrence of crosslinking inhibition caused by these gases. It is noted that the above-mentioned magnesium oxide is also capable of functioning as an acid acceptor.

Examples of the hydrotalcite include one or at least two members selected from a Mg-Al hydrotalcite such as Mg_{4.5}Al₂(OH)₁₃CO₃·3.5H₂O, Mg_{4.5}Al₂(OH)₁₃CO₃, Mg₄Al₂(OH)₁₂CO₃·3.5H₂O, Mg₆Al₂(OH)₁₆CO₃·4H₂O, Mg₅Al₂(OH)₁₄CO₃·4H₂O and Mg₃Al₂(OH)₁₀CO₃·1.7H₂O.

Specific examples of the hydrotalcite include DHT-4A (registered trademark)-2 available from Kyowa Chemical Industry Co. Ltd.

In the medical rubber composition, when the hydrotalcite is used as the acid acceptor, the hydrotalcite is preferably used together with MgO. In this case, the amount of the hydrotalcite is preferably considered as the total amount of the acid acceptor (hydrotalcite and MgO). The total amount of the acid acceptor (hydrotalcite and MgO) is preferably 0.5 part by mass or more, more preferably 1 part by mass or more, and is preferably 15 parts by mass or less, more preferably 10 parts by mass or less, with respect to 100 parts by mass of (a) the base polymer component. If the total amount of the acid acceptor (hydrotalcite and MgO) falls within the above range, rusting of a mold or the like can be suppressed, and defects that the materials themselves become white spot of foreign material can be reduced.

The medical rubber composition according to the present invention may further contain (d) a filler. Examples of (d) the filler include an inorganic filler such as clay and talc, and a resin powder of an olefin resin, styrene elastomer or ultrahigh molecular weight polyethylene (UHMWPE). Among them, as the filler, the inorganic filler is preferable, clay or talc is more preferable. The filler functions as adjusting the rubber hardness of the medical rubber member, and also functions as a weight increasing material to reduce the production cost of the medical rubber component.

Examples of the clay include burned clay and kaolin clay. Specific examples of the clay include SILLITIN (registered trademark) Z available from Hoffmann Mineral Corporation, SATINTONE (registered trademark) W available from Engelhard Corporation, NN Kaolin Clay available from Tsuchiya Kaolin Industry Co. Ltd., and PoleStar 200R available from IMERYS Specialities Japan K.K.

Specific examples of the talc include Hytron A available from Takehara Kagaku Kogyo Co., Ltd., MICRO ACE (registered trademark) K-1 available from Nippon Talc Co. Ltd., and Mistron (registered trademark) Vapor available from IMERYS Specialities Japan K.K.

The amount of (d) the filler in the medical rubber composition according to the present invention is preferably appropriately set according to the desired rubber hardness or the like of the medical rubber component. The amount of (d) the filler in the medical rubber composition according to the present invention is, for example, preferably 5 parts by mass or more, more preferably 10 parts by mass or more, and even more preferably 20 parts by mass or more, and is preferably 200 parts by mass or less, more preferably 150 parts by mass or less, and even more preferably 100 parts by mass or less, with respect to 100 parts by mass of (a) the base polymer.

The medical rubber composition according to the present invention may further contain a coloring agent such as titanium oxide and carbon black, a lubricant such as stearic acid and low density polyethylene (LDPE), a processing aid, polyethylene glycol acting as a crosslinking activator, or the like, in an appropriate proportion.

The medical rubber composition according to the present invention preferably does not contain an oil component because (b) the liquid polymer contained therein is used instead of the oil component. In addition, the crosslinked product of the medical rubber composition according to the present invention differs from a dynamically crosslinked thermoplastic elastomer (TPV) obtained by dynamically crosslinking a mixture of a thermoplastic component and a rubber component with a crosslinking agent.

### <Preparation of medical rubber composition>

The medical rubber composition according to the present invention is obtained by kneading (a) the base polymer, (b) the liquid polymer, and other materials which are added where necessary. The kneading can be performed, for example, using an open roll, a closed kneader, or the like. The kneaded mixture is preferably molded into a ribbon shape, a sheet shape, a pellet shape, or the like, and is more preferably molded into the sheet shape.

A medical rubber component having a desired shape is obtained by press molding the kneaded mixture in the ribbon shape, sheet shape or pellet shape. The crosslinking reaction of the medical rubber composition proceeds during the pressing. The molding temperature is, for example, preferably 130 °C or more, more preferably 140 °C or more, and is preferably 200 °C or less, more preferably 190 °C or less. The molding time is preferably 2 minutes or more, more preferably 3 minutes or more, and is preferably 60 minutes or less, more preferably 30 minutes or less. The molding pressure is preferably 0.1 MPa or more, more preferably 0.2 MPa or more, and is preferably 10 MPa or less, more preferably 8 MPa or less.

The extra part is cut off and removed from the molded product obtained by the press molding to obtain a predetermined shape. The obtained molded product is washed, sterilized, dried and packed to produce the medical rubber component.

In addition, as conventionally done, a resin film may be laminated on and integrated with the medical rubber component according to the present invention. Examples of the resin film include a film of an inactive resin such as polytetrafluoroethylene (PTFE), tetrafluoroethylene-ethylene copolymer (ETFE), their modified products, and ultra-high density polyethylene (UHDPE).

The resin film may be integrated with the medical rubber component formed by press molding, for example, by press molding the resin film in a state of being laminating on the sheet shaped rubber composition.

Examples of the medical rubber component according to the present invention include a rubber stopper or sealing component of a container for various medical preparations such as a liquid preparation, a powder preparation and a freeze-dried preparation, a sliding or sealing component such as a rubber stopper for a vacuum blood collecting tube, a plunger stopper or a nozzle cap of a prefilled syringe.

Among them, the rubber hardness of the rubber stopper or sealing component for a vial, an infusion preparation container or the like is preferably 35 or more and is preferably 60 or less in Durometer type A hardness (Shore A hardness) measured in accordance with the measuring method described in "Durometer hardness, Part III, Measuring method of hardness, Vulcanized rubber and thermoplastic rubber" of Japan Industry Specification JIS K 6253-3: 2012.

In addition, the above Shore A hardness of a sliding or sealing component such as a plunger stopper or a nozzle cap of a prefilled syringe is preferably 40 or more and is preferably 70 or less.

The rubber hardness of the medical rubber component can be adjusted by varying the mixing ratio of the raw materials.

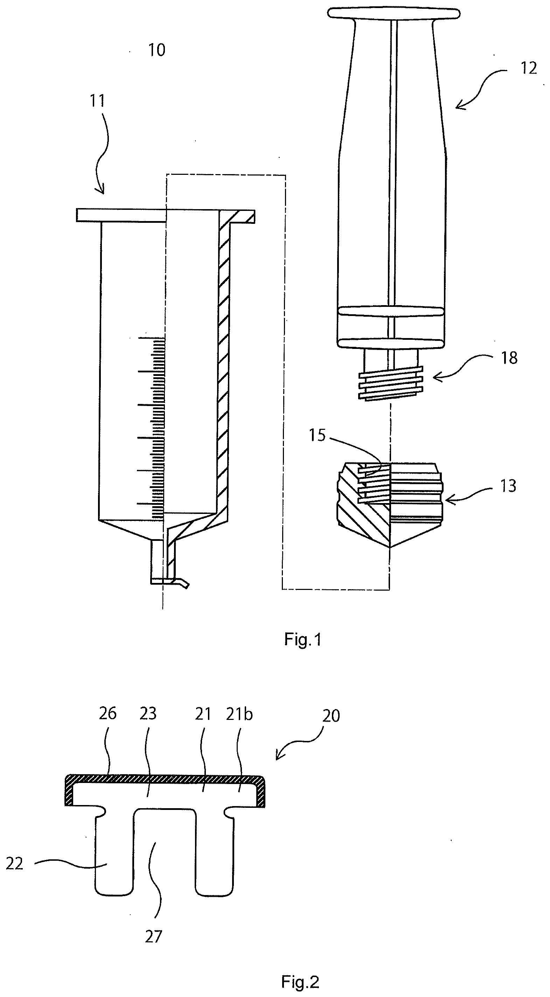

Fig. 1 is an exploded view showing a medical syringe using the medical rubber component according to the present invention, i.e. a syringe called a prefilled syringe. In Fig. 1, half of a syringe barrel 11 and a plunger stopper 13 are shown by a cross-section. The prefilled syringe 10 includes the cylindrical syringe barrel 11, a plunger 12 combined with the syringe barrel 11 and capable of reciprocating in the syringe barrel 11, and the plunger stopper 13 provided on the tip of the plunger 12. The plunger stopper 13 is molded from the medical rubber composition according to the present invention. A laminating film for improving slidability may be laminated on the surface of the plunger stopper 13.

The plunger 12 is, for example, composed of a resin plates having a cross-shape in a transverse sectional view and has a head portion 18 on the tip of the plunger 12. The plunger stopper 13 is connected to the head portion 18. The head portion 18 is integrally formed with the plunger 12, made of a resin, and processed into a male screw shape. The plunger stopper 13 has a roughly columnar shape with a short axis, and the tip surface of the plunger stopper 13, for example, has a mountain shape with an obtuse angle where the central axis of the columnar shape is protruding. Further, the plunger stopper 13 has a fitting recessed portion 15 in a female screw shape engraved in the axis direction thereof from the rear surface thereof. The head portion 18 of the plunger 12 is screwed into the fitting recessed portion 15 of the plunger stopper 13, to assemble the plunger stopper 13 on the tip of the plunger 12.

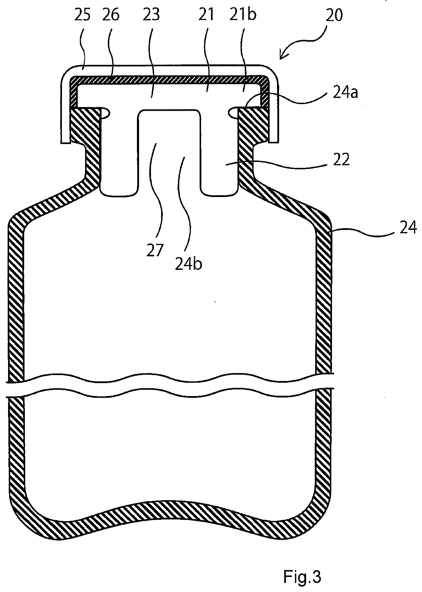

Fig. 2 is a schematic cross-sectional view of one specific example of a medical rubber stopper 20 that is the medical rubber component according to the present invention. Fig. 3 shows the medical rubber stopper 20 in a state that an opening 24b of a container 24 having a pharmaceutical product filled therein is sealed with a medical rubber stopper 20.

The medical rubber stopper 20 comprises a top plate 21 and a stopper leg 22. The top plate 21 has a penetration portion 23 through which a syringe needle of a syringe can be penetrated, and a flange 21b contacting the upper edge surface 24a of the container mouth of the medical container 24. The stopper leg 22 protrudes from the lower surface of the top plate 21, and is inserted in the mouth of the medical container. Further, the stopper leg 22 has a roughly cylindrical shape, and has a cut out space 27. A nylon film layer 26 is provided on the top face of the top plate 21 of the medical rubber stopper 20. If the nylon film layer 26 is provided on the top face of the medical rubber stopper 20, the mechanical transportability when manufacturing the pharmaceutical product can be ensured. Further, the nylon film layer 26 is provided on the top face of the medical rubber stopper 20, the surface smoothness of the top face can be improved, and occurrence of needle penetration fragment when penetrating the syringe needle can be prevented.

In the embodiment shown in Fig. 3, a vial storing a freeze-dried preparation is used as the container 24. In a case that the liquid pharmaceutical product to be kept in the container 24 is a pharmaceutical liquid for a syringe, the needle of the syringe is penetrated through the penetration portion 23 of the top plate 21, to suck the pharmaceutical liquid for syringe into the syringe without opening the medical rubber stopper 20. As such, the medical rubber stopper 20 is not opened to avoid a foreign material from entering the pharmaceutical liquid for syringe in the container 24.

A metallic or resinous cap 25 capable of covering the opening 24b of the container 24 and the medical rubber stopper 20 is provided on the top plate 21. Sealing the medical rubber stopper 20 and the opening 24b with the cap 25 is to prevent mycete from adhering to the penetration portion 23 at the site where the needle of the syringe is penetrated and prevent mycete from entering the pharmaceutical liquid for syringe through the syringe needle. Examples of the type of the cap 25 include a flip-off cap, a pull-top cap, and a clean cap. In a case of a hospital where a large amount of a pharmaceutical liquid for syringe is used, the clean cap which can be opened with one hand and provides easy handling is preferably used.

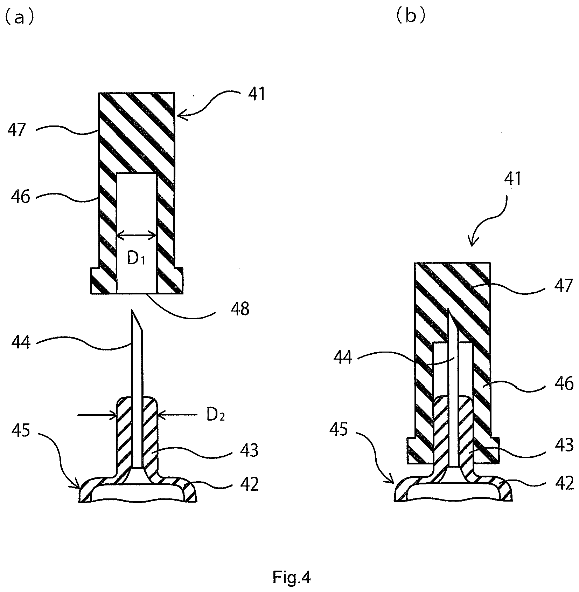

Fig. 4(a) is a cross-sectional view showing one example of a nozzle cap of a medical syringe and a nozzle of a syringe barrel. The nozzle is covered with the nozzle cap. Fig. 4(b) is a cross-sectional view showing a state of covering the nozzle with the nozzle cap. The nozzle cap 41 in this example is used for a needle-attached syringe 45 where the needle 44 is buried into the nozzle 43 of the syringe barrel 42 in advance. The nozzle cap is integrally formed from the medical rubber composition according to the present invention. The nozzle cap comprises a cylindrical portion 46 having an inner diameter D1 slightly less than the outer diameter D2 of the nozzle 43, and a needle penetration portion 47 connecting to one end side (upper end side in the figure) of the cylindrical portion 46. The needle penetration portion 47 is formed into a columnar shape having an outer surface continuous to the cylindrical portion 46. An opening 48 for inserting the nozzle 43 in the cylindrical portion 46 and covering the nozzle 43 with the nozzle cap 41, is formed on another end side (lower end side in the figure) of the cylindrical portion 46.

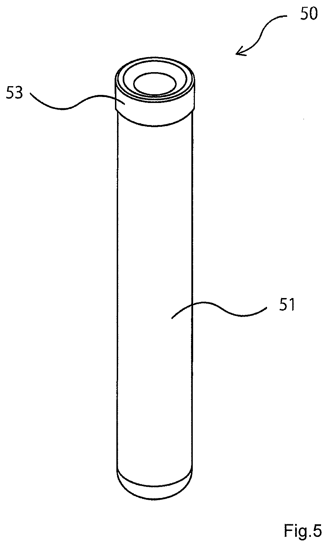

Fig. 5 is an illustrating figure showing one example of a vacuum blood collecting tube. The vacuum blood collecting tube 50 is composed of a tube 51 with bottom and a rubber stopper 53 sealing the opening of the tube 51 with bottom. The rubber stopper 53 is formed from the medical rubber composition according to the present invention. It is designed that blood can be automatically collected by reducing pressure in the blood collecting tube.

### EXAMPLES

Next, the present invention will be described in detail by way of examples. However, the present invention is not limited to the examples described below.

### [Preparation of medical rubber composition]

Materials shown in Table 1 were kneaded to prepare medical rubber compositions. The kneading was performed at 20 °C for about 10 minutes using an open roll. Rubber compositions 1 and 2 are reference compositions.

**Table 1**

| Materials | | Rubber composition | | | | | |
|---|---|---|---|---|---|---|---|
| | | 1 | 2 | 3 | 4 | 5 | 6 |
| Component (a) | Chlorinated butyl rubber | - | 100 | 100 | 100 | 100 | 100 |
| | Brominated butyl rubber | 100 | - | - | - | - | - |
| Component (b) | Liquid polybutene 1 | - | - | 10 | - | - | - |
| | Liquid polybutene 2 | - | - | - | 10 | - | - |
| | Liquid polybutene 3 | - | - | - | - | 10 | - |
| | Liquid polybutene 4 | - | - | - | - | - | 10 |
| | Liquid polybutene 5 | - | - | - | - | - | - |
| | Liquid polybutadiene | - | - | - | - | - | - |
| Component (c) | Triazine derivative | - | 1 | 1 | 1 | 1 | 1 |
| | Sulfur | 0.5 | - | - | - | - | - |
| Others | Hydrotalcite | - | 2 | 2 | 2 | 2 | 2 |
| | Magnesium oxide | 7 | 2 | 2 | 2 | 2 | 2 |
| | Titanium oxide | 3.9 | 3.9 | 3.9 | 3.9 | 3.9 | 3.9 |
| | Talc | 80 | 80 | 80 | 80 | 80 | 80 |
| | Carbon | 0.35 | 0.35 | 0.35 | 0.35 | 0.35 | 0.35 |
| | Oil | 10 | 10 | - | - | - | - |
| Total amount (parts by mass) | | 201.75 | 199.25 | 199.25 | 199.25 | 199.25 | 199.25 |
| Gas permeability | N₂ | 6.40 | 6.52 | 5.40 | 5.21 | 4.97 | 4.85 |
| | | E-11 | E-11 | E-11 | E-11 | E-11 | E-11 |
| | O₂ | 2.05 | 2.07 | 1.81 | 1.72 | 1.55 | 1.52 |
| | | E-10 | E-10 | E-10 | E-10 | E-10 | E-10 |
| | Air (N₂+O₂) | 9.93 | 1.01 | 8.58 | 8.21 | 7.59 | 7.44 |
| | | E-11 | E-10 | E-11 | E-11 | E-11 | E-11 |
| | Relative evaluation | 100 | 101 | 86 | 83 | 76 | 75 |
| | | P | P | G | G | E | E |
| Potassium permanganate reducing substance | | 0.2 | 0.1 | 0.2 | 0.2 | 0.1 | 0.1 |
| | | G | G | G | G | G | G |
| Processability | | G | G | G | G | G | G |

| | | | | | | | |
|---|---|---|---|---|---|---|---|
| Formulation: parts by mass, Gas permeability: cc·cm/cm²·sec·cmHg | | | | | | | |

**Table 1 - continued**

| Materials | | Rubber composition | | | | | |
|---|---|---|---|---|---|---|---|
| | | 7 | 8 | 9 | 10 | 11 | 12 |
| Component (a) | Chlorinated butyl rubber | 100 | 100 | 100 | 100 | 100 | - |
| | Brominated butyl rubber | - | - | - | - | - | 100 |
| Component (b) | Liquid polybutene 1 | - | - | - | - | - | - |
| | Liquid polybutene 2 | - | - | 2 | 5 | 15 | 10 |
| | Liquid polybutene 3 | - | - | - | - | - | - |
| | Liquid polybutene 4 | - | - | - | - | - | - |
| | Liquid polybutene 5 | 10 | - | - | - | - | - |
| | Liquid polybutadiene | - | 10 | - | - | - | - |
| Component (c) | Triazine derivative | 1 | 1 | 1 | 1 | 1 | - |
| | Sulfur | - | - | - | - | - | 0.5 |
| Others | Hydrotalcite | 2 | 2 | 2 | 2 | 2 | - |
| | Magnesium oxide | 2 | 2 | 2 | 2 | 2 | 7 |
| | Titanium oxide | 3.9 | 3.9 | 3.9 | 3.9 | 3.9 | 3.9 |
| | Talc | 80 | 80 | 80 | 80 | 80 | 80 |
| | Carbon | 0.35 | 0.35 | 0.35 | 0.35 | 0.35 | 0.35 |
| | Oil | - | - | - | - | - | - |
| Total amount (parts by mass) | | 199.25 | 199.25 | 191.25 | 194.25 | 204.25 | 201.75 |
| Gas permeability | N₂ | 4.96 | 4.63 | 3.88 | 4.04 | 5.76 | 5.25 |
| | | E-11 | E-11 | E-11 | E-11 | E-11 | E-11 |
| | O₂ | 1.58 | 1.47 | 1.25 | 1.38 | 1.82 | 1.76 |
| | | E-10 | E-10 | E-10 | E-10 | E-10 | E-10 |
| | Air (N₂+O₂) | 7.67 | 7.15 | 6.04 | 6.48 | 8.87 | 8.34 |
| | | E-11 | E-11 | E-11 | E-11 | E-11 | E-11 |
| | Relative evaluation | 77 | 72 | 61 | 65 | 89 | 84 |
| | | E | E | E | E | G | G |
| Potassium permanganate reducing substance | | 0.1 | 0.2 | 0.1 | 0.1 | 0.2 | 0.2 |
| | | G | G | G | G | G | G |
| Processability | | G | G | G | G | G | G |

| | | | | | | | |
|---|---|---|---|---|---|---|---|
| Formulation: parts by mass, Gas permeability: cc·cm/cm²·sec·cmHg | | | | | | | |

Details of the materials used above are as follows.
Chlorinated butyl rubber: HT-1066 available from Exxon Mobil Corporation
Brominated butyl rubber: X_Butyl BB 2030 available from LANXESS Co. Ltd.
Liquid polybutene 1: Polybutene HV35 (dynamic viscosity at 100 °C: 85 mm²/s) available from JXTG Energy Corporation
Liquid polybutene 2: Polybutene HV50 (dynamic viscosity at 100 °C: 110 mm²/s) available from JXTG Energy Corporation
Liquid polybutene 3: Polybutene HV100 (dynamic viscosity at 100 °C: 220 mm²/s) available from JXTG Energy Corporation
Liquid polybutene 4: Polybutene HV300 (dynamic viscosity at 100 °C: 590 mm²/s) available from JXTG Energy Corporation
Liquid polybutene 5: Polybutene Indopol H-100 (dynamic viscosity at 100 °C: 170 mm²/s) available from Ineos Co. Ltd.
Liquid polybutadiene: Ricon 131 (Brookfield type viscosity: 2750 mPa·s (25 °C)) available from Cray Valley Corporation
Hydrotalcite: ALCAMIZER 1 available from Kyowa Chemical Industry Co. Ltd.
Magnesium oxide: MAGSARAT 150s available from Kyowa Chemical Industry Co. Ltd.
Titanium oxide: KR-380 available from Titan Kogyo, Ltd.
Talc: Mistron Vapor available from IMERYS Specialities K.K
Carbon black: Diablack G available from Mitsubishi Chemical Corporation
Oil: PW380 available from Idemitsu Kosan Co., Ltd.
Triazine derivative: Disnet DB available from Sankyo Kasei Sangyo Co., Ltd.
Sulfur: sulfur including 5% of oil available from Tsurumi Chemical Industry Co., Ltd.

### [Evaluation method]

### (1) Gas permeation coefficient (cc·cm/cm²·sec·cmHg)

A testing piece for measuring gas permeation coefficient was prepared from the obtained medical rubber composition, and the gas permeation coefficient was measured. The measurement was performed according to JIS-K 6275-1 by a differential pressure method, using GTR-30XASR available from GTR Corporation.
Testing gas: N₂, O₂, air (75% of N₂ + 25% of O₂)
Testing sample: A slab with a thickness of from 0.5 mm to 1.0 mm was prepared by press molding the rubber composition at the temperature of 180 °C for 6 minutes.

### <Relative evaluation>

The rubber composition No.1 was adopted as a standard formulation and the gas permeation coefficient thereof was defined as 100, and the gas permeation coefficient of each rubber composition was represented by converting the gas permeation coefficient of each rubber composition into this index.
E(Excellent): 80 or less
G(Good): more than 80 and not less than 90
P(Poor): more than 90

### (2) Eluting material test

Testing sample: A slab with a thickness of 2 mm was prepared by press molding the above-blended rubber composition at a temperature of 180 °C for 6 minutes. The slab was punched with a punch having φ17 mm to obtain a testing sample.

Regarding the produced sample, "Eluting Material Test" described in "7.03 Test method for an infusion rubber stopper" of Japanese Pharmacopoeia, 17^{th} Edition, was performed. The optimized conditions were as follows.
State of testing liquid: colorless and transparent
UV transmittance: transmittances in a layer length of 10 mm at a wave length of 430 nm and a wave length of 650 nm were not lower than 99.0%.
Ultraviolet absorption spectrum: absorption at a wave length of 220 nm to 350 nm was not higher than 0.20.
pH: difference between the testing liquid and a blank testing liquid was not higher than 1.0.
Zinc: absorption of the testing liquid was not higher than that of the standard liquid.
Potassium permanganate reducing substance: not higher than 2.0 mL/100 mL (Specification of Japanese Pharmacopoeia)
Evaporation residue: not higher than 2.0 mg

In the Table, it is described as "G(Good)" when the concentration of potassium permanganate reducing substance was not higher than 0.5 mL/100 mL, and it is described as "F(Fair)" when the concentration of potassium permanganate reducing substance was higher than 0.5 mL/100 mL.

### (3) Processability of liquid polymer

G(Good): The liquid polymer has a low viscosity, and is easily handled.
P(Poor): The liquid polymer has a high viscosity, and is hardly handled.

The gas permeation coefficient and results of the eluting material test are shown in Tables 1 and 2.

It is apparent from Table 1 that the medical rubber component formed from the medical rubber composition according to the present invention has excellent gas permeation resistance.

This application is based on Japanese Patent application No. 2019-209030 filed on November 19, 2019.

## Claims

1. A medical rubber composition containing (a) a base polymer containing a halogenated butyl rubber, and (b) a liquid polymer,
wherein the medical rubber composition contains (b) the liquid polymer in an amount of from 1 part by mass to 18 parts by mass with respect to 100 parts by mass of (a) the base polymer and
wherein (b) the liquid polymer contains a liquid polybutene or a liquid polybutadiene.

2. The medical rubber composition according to claim 1, wherein (a) the halogenated butyl rubber includes a chlorinated butyl rubber or a brominated butyl rubber.

3. The medical rubber composition according to claim 1 or 2, wherein (b) the liquid polymer has a dynamic viscosity (100 °C) of from 50 mm²/s to 4000 mm²/s.

4. The medical rubber composition according to any one of claims 1 to 3, further containing (c) a crosslinking agent.

5. The medical rubber composition according to claim 4, wherein (a) the halogenated butyl rubber contains a chlorinated butyl rubber, and (c) the crosslinking agent contains a triazine derivative.

6. The medical rubber composition according to claim 4, wherein (a) the halogenated butyl rubber contains a brominated butyl rubber, and (c) the crosslinking agent contains a metal oxide.

7. The medical rubber composition according to any one of claims 1 to 6, further containing (d) a filler.

8. A medical rubber component formed from the medical rubber composition according to any one of claims 1 to 7.

9. The medical rubber component according to claim 8, wherein the medical rubber component is a cap or a plunger stopper of a syringe, or a rubber stopper for a vacuum blood collecting tube.

## Patentansprüche

1. Medizinische Kautschukzusammensetzung, enthaltend (a) ein Basispolymer, welches einen halogenierten Butylkautschuk enthält, und (b) ein flüssiges Polymer,
wobei die medizinische Kautschukzusammensetzung (b) das flüssige Polymer in einer Menge von 1 Massenteil bis 18 Massenteile bezogen auf 100 Massenteile des (a) Basispolymers enthält, und
wobei (b) das flüssige Polymer ein flüssiges Polybuten oder ein flüssiges Polybutadien enthält.

2. Medizinische Kautschukzusammensetzung nach Anspruch 1, wobei (a) der halogenierte Butylkautschuk einen chlorierten Butylkautschuk oder einen bromierten Butylkautschuk umfasst.

3. Medizinische Kautschukzusammensetzung nach Anspruch 1 oder 2, wobei (b) das flüssige Polymer eine dynamische Viskosität (100 °C) von 50 mm²/s bis 4000 mm²/s aufweist.

4. Medizinische Kautschukzusammensetzung nach einem der Ansprüche 1 bis 3, die zudem (c) ein Vernetzungsmittel enthält.

5. Medizinische Kautschukzusammensetzung nach Anspruch 4, wobei (a) der halogenierte Butylkautschuk einen chlorierten Butylkautschuk enthält, und (c) das Vernetzungsmittel ein Triazinderivat enthält.

6. Medizinische Kautschukzusammensetzung nach Anspruch 4, wobei (a) der halogenierte Butylkautschuk einen bromierten Butylkautschuk enthält, und (c) das Vernetzungsmittel ein Metalloxid enthält.

7. Medizinische Kautschukzusammensetzung nach einem der Ansprüche 1 bis 6, die zudem (d) einen Füllstoff enthält.

8. Medizinische Kautschukkomponente, gebildet aus der medizinischen Kautschukzusammensetzung nach einem der Ansprüche 1 bis 7.

9. Medizinische Kautschukkomponente nach Anspruch 8, wobei die medizinische Kautschukkomponente eine Kappe oder ein Kolbenstopfen einer Spritze, oder ein Kautschukstopfen für ein Vakuum-Blutsammelröhrchen ist.

## Revendications

1. Composition de caoutchouc médical contenant (a) un polymère de base contenant un caoutchouc de butyle halogéné, et (b) un polymère liquide, ladite composition de caoutchouc médical contenant (b) le polymère liquide dans une quantité comprise entre 1 partie en masse et 18 parties en masse par rapport à 100 parties en masse du (a) polymère de base et (b) le polymère liquide contenant un polybutène liquide ou un polybutadiène liquide.

2. Composition de caoutchouc médical selon la revendication 1, dans laquelle (a) le caoutchouc de butyle halogéné comprend un caoutchouc de butyle chloré ou un caoutchouc de butyle bromé.

3. Composition de caoutchouc médical selon la revendication 1 ou 2, dans laquelle (b) le polymère liquide présente une viscosité dynamique (100 °C) de 50 mm²/s à 4000 mm²/s.

4. Composition de caoutchouc médical selon l'une quelconque des revendications 1 à 3, contenant en outre (c) un agent de réticulation.

5. Composition de caoutchouc médical selon la revendication 4, dans laquelle (a) le caoutchouc de butyle halogéné contient un caoutchouc de butyle chloré, et (c) l'agent réticulant contient un dérivé de triazine.

6. Composition de caoutchouc médical selon la revendication 4, dans laquelle (a) le caoutchouc de butyle halogéné contient un caoutchouc de butyle bromé, et (c) l'agent de réticulation contient un oxyde métallique.

7. Composition de caoutchouc médical selon l'une quelconque des revendications 1 à 6, contenant en outre (d) une charge.

8. Composant de caoutchouc médical formé à partir de la composition de caoutchouc médical selon l'une quelconque des revendications 1 à 7.

9. Composant de caoutchouc médical selon la revendication 8, dans lequel le composant de caoutchouc médical est un capuchon ou une butée de piston d'une seringue, ou une butée en caoutchouc pour un tube de prélèvement sanguin sous vide.
